# EUROPEAN PATENT SPECIFICATION

(11) **EP 0 213 029 B1**
(45) Date of publication and mention of the grant of the patent: **15.09.1993**
(21) Application number: 86401710.8
(22) Date of filing: 31.07.1986
(51) Int. Cl.: C12N 1/38

(54) **Increased fermentation yield of expressed protein**
Fermentationsausbeute an exprimiertem Protein
Rendement de fermentation en protéine exprimée

(30) Priority: 02.08.1985 US 762000
(43) Date of publication of application: 04.03.1987
(73) Proprietor: Merck & Co., Inc., Rahway New Jersey 07065-0900 (US)
(72) Inventor: Carty, Christine E., Lansdale Pennsylvania 19446 (US)
(74) Representative: Warcoin, Jacques

(56) References cited:
- EP-A- 0 128 743
- EP-A- 0 201 239
- GENE, vol. 24, 1983, pages 289-297, Elsevier Science Publishers; P.P. STEPIEN et al.: "Synthesis of a human insulin gene. VI. Expression of the synthetic proinsulin gene in yeast"

## Description

### BACKGROUND OF THE INVENTION

The use of transformed eucaryotic hosts to express a desired foreign protein is known in the art through the use of recombinant DNA techniques. The transformed cells containing a vector having DNA coding for the desired foreign protein are then grown in cell culture using conventional techniques and the foreign protein, if secreted by the transformed host, is isolated from the cell culture fluids or, if not secreted, is isolated by rupturing the cells.

A modification of the fermentation techniques is to employ a vector which does not produce the desired foreign protein until a specific nutrient is added to the culture medium. Galactose is such a nutrient medium which can be used to induce expression of the desired foreign protein using techniques known to the art (Stepien et al., Gene 24: 289-298, 1983). While the galactose induction system works well to initiate expression of the desired foreign protein, it would be desirable to obtain the desired protein in higher yield.

The earlier European patent application EP-A-0 201 239 (published on 12.11.86) discloses a method in which galactose-inducible yeast cells which are transformed to produce a foreign protein are first grown in galactose-free medium. When the cells are at late stationary phase, the culture medium is removed and the washed cells are cultured in fresh medium containing galactose.

### OBJECTS OF THE INVENTION

It is, accordingly, an object of the present invention to provide a process for obtaining a desired foreign protein, namely HBsAg, in increased yield in a galactose-induced fermentation process.

### SUMMARY OF THE INVENTION

A galactose-regulated yeast strain expresses HBsAg in improved yield by growing the transformed yeast cells in culture and replacing the culture medium with fresh medium before adding the galactose to induce expression of the foreign protein.

### DETAILED DESCRIPTION

According to the present invention, eucaryotic cells are transformed by insertion of a vector containing DNA constructed to express HBsAg when induced by galactose. The vector also contains DNA coding for the production of an essential nutrient, e.g., leucine, uracil, or tryptophan which is not made by non-transformed host cells. This assures that only host cells which retain the recombinant plasmid vector will reproduce as any cells which lose the plasmid will die. The recombinant eucaryotic cells, e.g. Saccharomyces cerevisiae, are used to inoculate a leucine and galactose deficient culture medium, e.g., an agar slant.

The host cells are then grown in culture according to techniques suitable for culture of the particular cells chosen to express the desired end product. When the cell density is at or about its maximum and the cells are in late log/early stationary phase, the cells are placed in fresh cell culture medium containing galactose to induce expression. The cells then are incubated for 2 to 3 cell doublings, to assure maximum expression of product. Optionally, but preferably the cells are washed before being placed in fresh cell culture medium. Washing may be done with saline, phosphate buffered saline or other physiologically acceptable solutions.

The desired protein may be secreted into the medium in which case a synthetic culture medium is used to facilitate recovery of the desired protein. If the desired protein is not secreted into the medium, it is obtained by rupturing the cells at temperatures of from about 2° to about 8°C and isolating the desired protein by any suitable means.

While the present invention is exemplified in the following description with reference to a specific yeast strain, it is to be understood that the invention may be practiced equally as well with other yeast strains.

The following examples illustrate the present invention without, however, limiting the same thereto.

### EXAMPLE 1

The Gal4 regulatory region between the Gal1 and Gal10 genes of yeast Saccharomyces cerevisiae was isolated as a 370 bp XhoI-Sau3A fragment from plasmid plGSD5, Guarente et al., Proc. Natl. Acad. Sci., USA 79:7410-7414 (1982). This fragment was converted by standard techniques to an -370 bp BamHl-Sall fragment and cloned as a BamHl-Sall substitution into the yeast expression vector pCl/l, Brake et al., Proc. Natl. Acad. Sci., USA 81:4642-4646 (1984) to give plasmid pCl/lGAL4/370. The -1.6 kb expression cassette containing 376 bp of GAPDH promoter sequence, Holland et al., J. Biol. Chem. 254: 9839-9845 (1979) and Travis et al., J. Biol. Chem. 260:4384-4389 (1985), the sequence for the entire coding region of the mature hepatitis B surface antigen and 128 bp of 3' untranslated sequence, Valenzuela et al., in Fields, Jaenisch and Fox (eds.), Animal Virus Genetics, Academic Press, New York, 1980, as well as the 350 bp ADHl terminator, Bennetzen et al., J. Biol. Chem. 257:3018-3025, (1982), was constructed as a SalI fragment and cloned into the SalI site of pCl/lGAL4/370. A plasmid, pHBSGAP80GAL370/2 was obtained which had the expression cassette in the desired orientation relative to the GAL regulatory region. The restriction map of this plasmid is as follows: 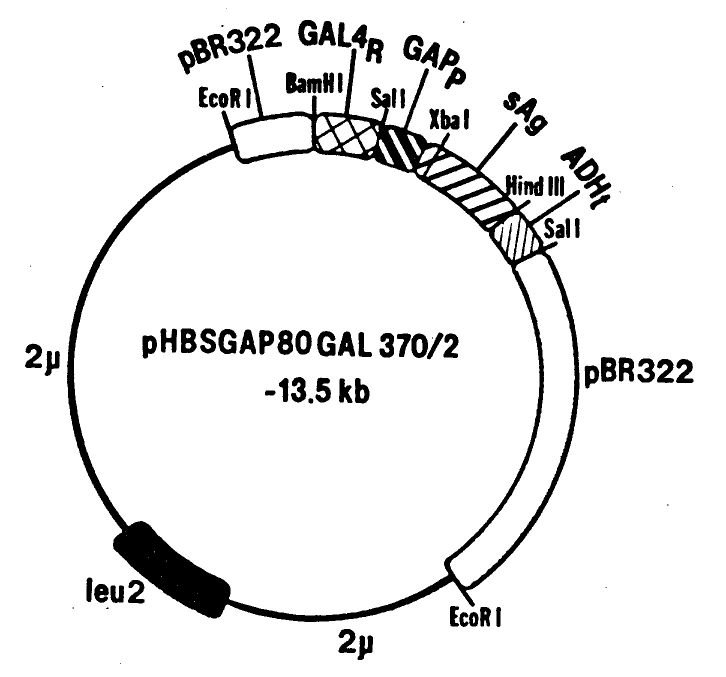
pBR322 GAL4R GAPP
EcoRI
BamHI
SalI
XbaI
sAg
ADHt
HindIII
SalI
pHBSGAP80GAL 370/2
-13.5 kb
2μ
pBR322
leu2
EcoRI
2μ
Yeast strain 2150-2-3 (Mata, leu2, adel, nib6⁺, [cir°], derived from a genetic cross between strain Y379-5-D cyh2 nibl (rho⁻), Livingston, Genetics 86:73 (1977), and DC O4 a Adel, Adex, leu2-04 (cir°), Broach, Cell 21:501 (1980), was transformed with pHBSGAP80GAL370/2 essentially according to Hinnen et al., Proc. Natl. Acad. Sci., USA 75: 1919-1933 (1978), to give tranformed yeast strain p5. Leu⁺ transformants were selected and maintained in synthetic complete medium lacking leucine (SC-leu). SC-leu medium contained 0.67% yeast nitrogen base without amino acids; 1% succinic acid; 0.6% NaOH; 2% glucose; 100 µg/ml adenine; 30 µg/ml each of uridine, tyrosine and lysine; 20 µg/ml each of tryptophan, histidine, arginine and methionine; and 200 µg/ml of threonine.

### EXAMPLE 2

A. A leucine and galactose deficient agar slant was inoculated with 1 ml of reconstituted lyophilized recombinant Saccharomyces cerevisiae cells, strain P5, which expresses the HBsAg gene when induced with galactose. After 4 days at 28°C the slant was resuspended in 5 ml yeast extract-peptone-dextrose (YEPD) medium and a 250 ml flask containing 46 ml of uninoculated YEPD medium was inoculated with 4 ml of the cell suspension. The flask was incubated at 28°C in a shaker/incubator at 350 rpm for 7.5 hours. The contents of the 250 ml flask were transferred to a two liter flask containing 450 ml of uninoculated YEPD medium and the incubation was continued for 19 hours.
This 500 ml yeast culture (O.D. of 7.5 at 660 nm) was added to a 16 L New Brunswick fermenter containing 5.0L of uninoculated YEPD medium. The fermentation was continued at 28°C, stirred initially at 300 rpm and sparged with air at a rate of 3 L/minute. Throughout the fermentation dissolved oxygen was controlled at approximately 10% of saturation and the pH at 5.0. After cell growth began to increase, YEPD medium was pumped into the fermenter from the 18th to the 52nd hour of the fermentation at the following rates:

| Hours | Rates (ml/hour) |
|---|---|
| 18-27 | 60 |
| 27-29.5 | 130 |
| 29.5-52 | 180 |

At hour 52 cell density was at a maximum and the cells were in late log/early stationary phase.
B. Approximately 1.0 L of yeast culture was removed at 52 hours. Half of the cell broth (500 ml) was transferred to a 2L flask; galactose was added to a final concentration of 2% (w/v). The flask was incubated at 28°C in a shaker/incubator at 350 rpm for 20 hours.
Cell growth of the culture was estimated by determination of O.D. at 660 nm and by measurement of dry weight. The O.D. was 36 and the dry weight was 34.2 g/L. The cells were then ruptured at 4°C by shearing with glass beads. The HBsAg concentration was less than 0.0001 mg/L by AUSRIA.
C. The other 500 ml of fermenter culture was centrifuged at 10,000 x g for 20 minutes. The pelleted cells were resuspended in 400 ml phosphate-buffered saline (PBS) and centrifuged again at 10,000 x g for 20 minutes to remove entrained medium components. The pelleted cells were resuspended again in 400 ml of PBS and centrifuged a third time at 10,000 x g for 20 minutes. The pelleted yeast cells were then resuspended in 500 ml yeast extract-peptone, galactose was added to a final concentration of 2% (w/v) and the culture was incubated at 28°C in a shaker/incubator at 350 rpm for 20 hours.
Cell growth of the culture was estimated by determination of O.D. at 660 nm and by measurement of dry weight. The O.D. was 44 and the dry weight was 35.1 g/L. The cells were then ruptured at 4°C by shearing with glass beads. The HBsAg concentration was 0.15 mg/L by AUSRIA.

### EXAMPLE 3

The procedure of part A of Example 1 is repeated. One liter of the yeast culture is then removed and filtered using a 0.45 µ filter. The yeast cells are then resuspended in 1.0 L yeast extract peptone and galactose is added to a final concentration of 2% (w/v). The culture is then incubated at 28°C in a shaker/incubator at 350 rpm for 20 hours. HBsAg is obtained in concentration similar to that of Example 1.

### EXAMPLE 4

The procedure of part A of Example 1 is repeated. One liter of the yeast culture is then removed and diafiltered against PBS. The yeast cells are then resuspended in 1.0 L yeast extract peptone and galactose is added to a final concentration of 2% (w/v). The culture is then incubated at 28°C in a shaker/incubator at 350 rpm for 20 hours. HBsAg is obtained in concentration similar to that of Example 1.

## Claims

1. A process for the production of recombinant HBsAg by yeast cells comprising :
(a) transforming yeast cells with DNA encoding HBsAg under transcriptional control of a galactose inducible promoter,
(b) culturing the transformed yeast cells in a medium substantially free of galactose,
(c) removing substantially all of the culture medium from the cultured yeast cells, when the cell density is at/or about its maximum and the cells are in late log/early stationary phase,
(d) adding fresh culture medium to the cultured yeast cells, and
(e) culturing the yeast cells in the presence of galactose to induce HBsAg expression.

2. The process according to Claim 1 wherein the galactose is present in the fresh culture medium of step (d).

3. The process according to Claim 1, wherein substantially all of the culture medium is removed from the yeast cells in step (c) before adding the fresh culture medium of step (d).

4. A process according to one of Claims 1 to 3, wherein the spent medium is removed by centrifugation.

5. A process according to one of Claims 1 to 3, wherein the spent medium is removed by filtration.

6. A process according to one of Claims 1 to 3, wherein the spent medium is removed by diafiltration.

## Patentansprüche

1. Verfahren zur Herstellung von rekombinierter HBsAg durch Hefezellen, welches umfaßt:
(a) Transformieren von Hefezellen mit DNA kodierend HBsAg unter Transkriptionskontrolle eines mit Galactose induzierbaren Promotors,
(b) Kultivieren der transformierten Hefezellen in einem im wesentlichen Galactose-freiem Medium,
(c) Entfernen im wesentlichen des gesamten Kulturmediums aus den Hefezellen, wenn die Zelldichte an oder bei ihrem Maximum ist und die Zellen in einer späten Log/frühen stationären Phase sind,
(d) Zufügen von frischem Kulturmedium zu den kultivierten Hefezellen, und
(e) Kultivieren der Hefezellen in Gegenwart von Galactose, um HBsAg-Expression zu induzieren.

2. Verfahren nach Anspruch 1, worin die Galactose im frischen Kulturmedium aus Schritt (d) vorliegt.

3. Verfahren nach Anspruch 1, worin im wesentlichen das gesamte Kulturmedium aus den Hefezellen in Schritt (c) vor dem Zugeben des frischen Kulturmediums entfernt wird.

4. Verfahren nach einem der Ansprüche 1 bis 3, worin das verbrauchte Medium durch Zentrifugieren entfernt wird.

5. Verfahren nach einem der Ansprüche 1 bis 3, worin das verbrauchte Medium durch Filtration entfernt wird.

6. Verfahren nach einem der Ansprüche 1 bis 3, worin das verbrauchte Medium durch Diafiltration entfernt wird.

## Revendications

1. Procédé de production d'antigène HBs recombinant par des cellules de levure, selon lequel :
(a) on transforme des cellules de levure avec un ADN codant pour l'antigène HBs à transcription régulée par un promoteur inductible par le galactose,
(b) on cultive les cellules de levure transformées dans un milieu à peu près exempt de galactose,
(c) on élimine presque la totalité du milieu de culture à partir des cellules de levure cultivées, lorsque la densité de cellules est égale ou voisine à sa valeur maximum et que les cellules sont en phase logarithimique tardive/stationnaire précoce,
(d) on ajoute un milieu de culture frais dans les cellules de levure cultivées, et
(e) on cultive les cellules de levure en présence de galactose pour induire l'expression de l'antigène HBs.

2. Procédé selon la revendication 1, dans lequel le galactose est présent dans le milieu de culture frais de l'étape (d).

3. Procédé selon la revendication 1, dans lequel presque la totalité du milieu de culture est éliminée à partir des cellules de levure dans l'étape (c), avant d'ajouter le milieu de culture frais de l'étape (d).

4. Procédé selon l'une des revendications 1 à 3, dans lequel le milieu usé est éliminé par centrifugation.

5. Procédé selon l'une des revendications 1 à 3, dans lequel le milieu usé est éliminé par filtration.

6. Procédé selon l'une des revendications 1 à 3, dans lequel le milieu usé est éliminé par diafiltration.
